**Europäisches Patentamt**

**European Patent Office**

**Office européen des brevets**

(11) Veröffentlichungsnummer: **0 434 035 A1**

## (12) EUROPÄISCHE PATENTANMELDUNG

(21) Anmeldenummer: **90124777.5**

(22) Anmeldetag: **19.12.90**

(51) Int. Cl.⁵: **C12P 17/12**, C12N 1/20,
//(C12N1/20,C12R1:38,1:07,
1:025)

Der (Die) Mikroorganismus (Mikroorganismen) ist (sind) bei DSM-Deutsche Sammlung von Mikroorganismen unter der Nummer DSM 2783 hinterlegt worden.

(30) Priorität: **20.12.89 CH 4555/89**

(43) Veröffentlichungstag der Anmeldung:
**26.06.91 Patentblatt 91/26**

(84) Benannte Vertragsstaaten:
**AT BE CH DE ES FR GB IT LI NL SE**

(71) Anmelder: **LONZA AG**
**Gampel/Wallis Geschäftsleitung Basel**
**CH-4002 Basel(CH)**

(72) Erfinder: **Hoeks, Frans, Dipl.-Ing.**
**Dammweg 11A**
**Naters (Kanton Wallis)(CH)**
Erfinder: **Venetz, Daniel**
**Rhonesandstrasse 26**
**Brig (Kanton Wallis)(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

(54) **Verfahren zur Herstellung von 6-Hydroxynikotinsäure.**

(57) Es wird ein Verfahren zur Herstellung von 6-Hydroxynikotinsäure aus Nikotinsäure durch enzymatische Hydroxylierung in Gegenwart von Mikroorganismen der Gattungen Pseudomonas, Bacillus oder Achromobacter beschrieben.

Durch Einhalten eines bestimmten Konzentrationsbereichs während der Nikotinsäurezugabe kann die Biomassebildung im selben Verfahrensschritt wie die Produktbildung erfolgen, ohne dass es zu Produktverlusten durch weiteren Abbau kommt.

EP 0 434 035 A1

## VERFAHREN ZUR HERSTELLUNG VON 6-HYDROXYNIKOTINSÄURE

Die Erfindung betrifft ein Verfahren zur Herstellung von 6-Hydroxynikotinsäure durch enzymatische Hydroxylierung von Nikotinsäure.

Es sind Verfahren bekannt, die die Herstellung von 6-Hydroxynikotinsäure mittels lebender Mikroorganismen der Gattungen Pseudomonas, Bacillus oder Achromobacter erlauben (CH-PS 658 866, CH-PS 658 867). Diese Verfahren verwenden eine Biomasse-Suspension der entsprechenden Mikroorganismen, welche in einem separaten Verfahrensschritt durch Vermehrung einer Starterkultur gewonnen wird. Die eigentliche Hydroxylierung erfolgt entweder in einem diskontinuierlichen Verfahren mit einmaliger Zugabe der Nikotinsäure als Natriumsalz (Batch-Verfahren, CH-PS 658 866) oder in einem kontinuierlichen Verfahren, bei dem die Nikotinsäure als (gut lösliches) Magnesium- oder Bariumsalz zugegeben und die 6-Hydroxynikotinsäure als schwerlösliches Magnesium- oder Bariumsalz isoliert wird (CH-PS 658 867). Da die Vermehrung der Mikroorganismen durch die bei diesen Verfahren angewandten Nikotinsäurekonzentrationen gehemmt wird, ist es bei beiden Verfahren erforderlich, die gesamte Menge an wirksamer Biomasse in einem vorgeschalteten Verfahrensschritt zu gewinnen. Ausserdem besitzen die bekannten Verfahren zur enzymatischen Herstellung von 6-Hydroxynikotinsäure noch weitere Nachteile. Das kontinuierliche Verfahren gemäss CH-PS 658 867 liefert Magnesium- oder Bariumsalze, aus denen die 6-Hydroxynikotinsäure durch Säurezusatz freigesetzt wird. Dabei entsteht das Magnesium- oder Bariumsalz der zugesetzten Säure, welches als Abfall entsorgt werden muss. Insbesondere lösliche Bariumsalze sind jedoch für höhere Organismen stark toxisch. Bei längerem kontinuierlichen Betrieb kommt es zudem leicht zu Verkrustungen durch die auskristallisierenden Salze oder zu Fremdinfektionen.

Aufgabe der Erfindung war daher die Bereitstellung eines einfacheren Verfahrens, das unter Vermeidung der genannten Nachteile 6-Hydroxynikotinsäure in hoher Konzentration und guter Ausbeute liefert.

Erfindungsgemäss wird diese Aufgabe durch ein Verfahren nach Patentanspruch 1 gelöst.

Es wurde nämlich überraschend gefunden, dass auf die separate Gewinnung der Biomasse verzichtet werden kann, wenn ausgehend von einer auf an sich bekannte Weise gewonnenen Starterkultur die Nikotinsäure derart zugegeben wird, dass die Nikotinsäurekonzentration in der Suspension der Mikroorganismen nach vollständiger Durchmischung unterhalb derjenigen Konzentration bleibt, oberhalb derer die Vermehrung der Mikroorganismen gehemmt wird. Dass unter diesen Bedingungen Biomasse gebildet wird, war zu erwarten, überraschend ist jedoch, dass während dieser wachstumsphase 6-Hydroxynikotinsäure produziertund nicht weiter metabolisiert wird, so dass nach Beendigung der Nikotinsäurezugabe und vollständigem Verbrauch der Nikotinsäure die 6-Hydroxynikotinsäure in praktisch quantitativer Ausbeute nach an sich bekannten Verfahren isoliert werden kann. Die während der Zugabe von Nikotinsäure steigende Konzentration von 6-Hydroxynikotinsäure hemmt dabei das Zellwachstum in zunehmendem Masse. Das Wachstum kommt jedoch erst bei relativ hohen Konzentrationen von 6-Hydroxynikotinsäure ganz zum Stillstand. Je nach eingesetztem Stamm des Mikroorganismus liegen diese im Bereich von 50 g/l oder darüber. Ueberraschenderweise findet dann auch der weitere Abbau von 6-Hydroxynikotinsäure nicht mehr statt. Eine Hemmung der Nikotinsäure-Hydroxylase wird jedoch erst bei noch höheren Konzentrationen beobachtet und begrenzt damit die erreichbare Produktkonzentration bei 100%iger Ausbeute. Wesentlich für das erfindungsgemässe Verfahren ist, dass die Nikotinsäurekonzentration während des Zellwachstums nie auf Null absinkt, da sonst die bereits gebildete 6-Hydroxynikotinsäure abgebaut wird. Erst wenn die maximale Konzentration an 6-Hydroxynikotinsäure nahezu erreicht ist, wird auch der weitere Abbau gehemmt, so dass nach Ende der Nikotinsäurezugabe ihr vollständiger Verbrauch abgewartet werden kann, ohne Ausbeuteverluste befürchten zu müssen. Als Nikotinsäure hydroxylierende Mikroorganismen werden vorteilhaft solche der Gattungen Pseudomonas, Bacillus oder Achromobacter eingesetzt. Bevorzugt sind dabei die Species Pseudomonas putida und Achromobacter xylosoxydans, besonders bevorzugt der Stamm Achromobacter xylosoxydans DSM 2783 (hinterlegt bei: Deutsche Sammlung von Mikroorganismen, Grisebachstr. 8, D-3400 Göttingen). Die bevorzugten Mikroorganismen sind inder CH-PS 658 866 beschrieben. Die Nikotinsäurekonzentration in der Suspension der Mikroorganismen liegt während der Zugabedauer vorzugsweise unterhalb von 10 g/l.

Die Zugabe der Nikotinsäure kann in kleinen Portionen oder kontinuierlich erfolgen, bevorzugt ist eine kontinuierliche Zugabe.

Die Nikotinsäure kann in fester oder gelöster Form zugegeben werden, und zwar jeweils als freie Säure oder als wasserlösliches Salz. Bevorzugt ist die Zugabe in wässriger Lösung, besonders bevorzugt als wässrige Lösung des Natrium- oder Kaliumsalzes.

Die Suspension der Mikroorganismen wird zweckmässig belüftet und gerührt, es ist auch möglich, die nötige Rührwirkung durch die eingeblasene Luft zu erzielen. Die Partialspannung des gelösten Sauerstoffs (pO₂) liegt dabei vorteilhaft zwischen 1 mbar und 200 mbar, vorzugsweise im Bereich 40-80 mbar. Bevorzugt ist die Verwendung eines mechanischen Rührers. Die Vermehrung der Mikroorganismen und die Bildung der 6-Hydroxynikotinsäure erfolgt vorzugsweise bei einer Temperatur von 20 bis 40 °C und einem pH von 5,5 bis 9.

Vorteilhaft werden zusammmen mit der Nikotinsäure weitere Nährstoffe für das Wachstum der Biomasse eingesetzt. Hierzu zählen Kohlenstoffquellen, wie beispielsweise Glycerin oder Glucose, Stickstoffquellen, wie beispielsweise Ammoniumsalze oder Glutaminsäure, sowie Mineralsalze, Spurenelemente und Vitamine. diese werden zweckmässig derart zugesetzt, dass ihre Konzentration in der Mikroorganismen-Suspension weder im limitierenden noch im hemmenden Bereich liegt. Diese Verfahrensweise ist dem Fachmann jedoch geläufig. Die Nährstoffe können sowohl einzeln als auch in komplexer Form als natürliche oder künstlich hergestellte Gemische zugesetzt werden. Ein besonders bevorzugter komplexer Nährstoff ist Hefeextrakt.

Das erfindungsgemässe Verfahren kann auch in kontinuierlicher Arbeitsweise durchgeführt werden, indem nach Erreichen einer Konzentration von 6-Hydroxynikotinsäure, die hoch genug ist, um einen weiteren Abbau zu anderen Produkten zu verhindern, mit einer Abtrennung des Produkts bei gleichzeitiger Zellrückführung begonnen wird. Eine dafür geeignete Apparatur ist beispielsweise in der CH-PS 664 374 beschrieben. Die Zugabegeschwindigkeit der Nikotinsäure wird dabei zweckmässig so gross wie die Geschwindigkeit der Produktentnahme gewählt, so dass die Produktkonzentration konstant bleibt.

Das nachfolgende Beispiel verdeutlicht die Durchführung des erfindungsgemässen Verfahrens.

Beispiel:
a) Herstellung der Starterkultur:
Aus 5,19 g Dinatriumhydrogenphosphat-Dihydrat, 2,00 g Kaliumdihydrogenphosphat, 0,25 g Hefeextrakt, 1,00 g Nikotinsäure und 500 ml Wasser wurde eine Nährlösung hergestellt und 20 Min. bei 120 °C sterilisiert. Nach Abkühlen auf 30 °C wurde eine sterile konzentrierte Spurenelement-Lösung in solcher Menge zugegeben, dass die nachfolgenden Konzentrationen im Kulturmedium erreicht wurden:

| | | |
|---|---|---|
| Calciumchlorid-Dihydrat | 20 | mg/l |
| Mangan(II)-sulfat | 10 | mg/l |
| Eisen(II)-sulfat-Heptahydrat | 5 | mg/l |
| Cobalt(II)-sulfat-Hexahydrat | 0,1 | mg/l |
| Kupfer(II)-sulfat-Pentahydrat | 0,1 | mg/l |
| Zinksulfat-Heptahydrat | 0,1 | mg/l |
| Natriummolybdat-Dihydrat | 0,1 | mg/l |

Das Kulturmedium wurde mit Achromobacter xylosoxydans DSM 2783 angeimpft und 24 h bei 30 °C und pH 7 kultiviert.

b) Herstellung von 6-Hydroxynikotinsäure:
In einem 20 l-Fermenter mit Rührer, Belüftung und pH-Regelung wurden in 12 l Wasser 90 g Nikotinsäure, 19,44 g Natriumhydroxid, 90 g Hefeextrakt, 12 g Kaliumsulfat, 9,6 g Magnesiumchlorid-Hexahydrat, 1,92 g Calciumchlorid, 2,4 ml Polypropylenglykol 2000, 180 g L-Glutaminsäure und 300 g Glucose gelöst und 30 Min. bei 121 °C sterilisiert. Nach Abkühlung auf 30 °C wurde die Starterkultur zugegeben und unter Luftzufuhr und Rühren bei pH 7 10 h kultiviert. Nach dieser Zeit war die Nikotinsäurekonzentration von 7,5 g/l auf 2 g/l abgesunken (HPLC-Bestimmung) und die Biomassekonzentration auf 10 g/l (Trockenmasse) angestiegen. Zu diesem Zeitpunkt wurde mit der kontinuierlichen Zugabe einer 20 Min. bei 121 °C sterilisierten Lösung von 1,13 kg Nikotinsäure und 0,365 kg Natriumhydroxid in 3,00 l Wasser begonnen, wobei die Zugabegeschwindigkeit so eingestellt wurde, dass die Nikotinkonzentration im Fermenter während der Zugabe zwischen 1 g/l und 9 g/l lag. Nach 11 h war die Zugabe beendet, Glutaminsäure und Glucose vollständig verbraucht und die Biomassekonzentration auf 15 g/l (Trockenmasse) gestiegen. Nach weiteren 4 h, also insgesamt 25 h nach der Inokulation wurde die Fermentation abgebrochen. Die Endkonzentration an 6-Hydroxynikotinsäure betrug 74 g/l, was einer praktisch quantitativen Umwandlung von Nikotinsäure in 6-Hydroxynikotinsäure entspricht. Die Aufarbeitung erfolgte wie in der CH-PS 658 866 (Beispiele 1 und 3) beschrieben.

## Ansprüche

1. Verfahren zur Herstellung von 6-Hydroxynikotinsäure durch mikrobiologische Hydroxylierung von Nikotinsäure unter aeroben Bedingungen, dadurch gekennzeichnet, dass Nikotinsäure oder ein lösliches Salz der Nikotinsäure oder eine Nikotinsäure enthaltende Lösung derart kontinuierlich oder portionsweise zu einer Starterkultur des Mikroorganismus zugegeben wird, dass die Nikotinsäurekonzentration in der Kultur während der Dauer der Zugabe stets grösser als Null ist und nach vollständiger Durchmischung wenigstens solange unterhalb der Konzentration bleibt, oberhalb welcher eine wachstumshemmung des Mikroorganismus eintritt, bis die gebildete 6-Hydroxynikotinsäure das weitere Wachstum hemmt, so dass die Vermehrung des Mikroorganismus und die Bildung von 6-Hydroxynikotinsäure im selben Verfahrensschritt erfolgen.

2. Verfahren nach Patentanspruch 1, dadurch gekennzeichnet, dass ein Mikroorganismus aus einer der Gattungen Pseudomonas, Bacillus oder Achromobacter verwendet wird.

3. Verfahren nach Patentanspruch 2, dadurch gekennzeichnet, dass ein Mikroorganismus aus einer der Species Pseudomonas putida und Achromobacter xylosoxydans verwendet wird.

4. Verfahren nach Patentanspruch 3, dadurch gekennzeichnet, dass ein Achromobacter xylosoxydans vom Stamm DSM 2783 verwendet wird.

5. Verfahren nach einem der Patentansprüche 1 bis 4, dadurch gekennzeichnet, dass die Nikotinsäurekonzentration in der Kultur während der Dauer der Zugabe unterhalb von 10 g/l gehalten wird.

6. Verfahren nach einem oder mehreren der Patentansprüche 1 bis 5, dadurch gekennzeichnet, dass die Nikotinsäure als Lösung kontinuierlich zu einer gerührten und belüfteten Suspension des Mikroorganismus zugegeben wird.

7. Verfahren nach einem oder mehreren der Patentansprüche 1 bis 6, dadurch gekennzeichnet, dass die Vermehrung des Mikroorganismus und die Bildung der 6-Hydroxynikotinsäure bei einer Temperatur von 20 bis 40°C und einem pH von 5,5 bis 9 erfolgt.

8. Verfahren nach einem oder mehreren der Patentansprüche 1 bis 7, dadurch gekennzeichnet, dass die Nikotinsäure in Form einer wässrigen Lösung ihres Natriumsalzes zusammen mit einem Nährstoff eingesetzt wird.

Europäisches
Patentamt

**EUROPÄISCHER
RECHERCHENBERICHT**

Nummer der Anmeldung

**EP 90 12 4777**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 152 948 (LONZA AG) <br> * das ganze Dokument * <br> – – – | 1-8 | C 12 P <br> 17/12 <br> C 12 N 1/20 // |
| D,Y | EP-A-0 152 949 (LONZA AG) <br> * das ganze Dokument * <br> – – – | 1-8 | (C 12 N 1/20; <br> C 12 R 1:38 <br> C 12 R 1:07 |
| A | BIOCHEM. BIOPHYS. ACTA vol. 9, 1952, Seiten 226 - 227; HUGHES, D.E.: "6-Hydroxy nicotinic acid as an intermediate in the oxydation of nicotinic acid by Pseudomonas fluorescens" <br> * das ganze Dokument * <br> – – – – – | 1 | C 12 R <br> C 12 R 1:025) |

|  |
|---|
| **RECHERCHIERTE SACHGEBIETE (Int. Cl.5)** |
| C 12 P <br> C 12 R |

Der vorliegende Recherchenbericht wurde für alle Patentansprüche erstellt

| Recherchenort | Abschlußdatum der Recherche | Prüfer |
|---|---|---|
| Den Haag | 12 März 91 | ANDRES S.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument
---------------------------------------------------------------
& : Mitglied der gleichen Patentfamilie, übereinstimmendes Dokument